# EUROPEAN PATENT SPECIFICATION

(11) **EP 2 861 260 B1**
(45) Date of publication and mention of the grant of the patent: **10.08.2016**
(21) Application number: 13731436.5
(22) Date of filing: 17.06.2013
(51) Int. Cl.: A61K 47/48, C08G 65/329

(54) **CONJUGATION REAGENTS**
KONJUGATIONSREAGENZIEN
RÉACTIFS DE CONJUGAISON

(30) Priority: 18.06.2012 GB 201210770
(43) Date of publication of application: 22.04.2015
(73) Proprietor: Polytherics Limited, Babraham Cambridge CB22 3AT (GB)
(72) Inventor: GODWIN, Antony, Cambridge, CB22 3AT (GB)
(74) Representative: Scott, Susan Margaret
(86) International application number: PCT/GB2013/051567
(87) International publication number: WO 2013/190272

(56) References cited:
- WO-A1-2009/047500
- US-B2- 7 985 838
- YUEHUA CONG ET AL: "Site-Specific PEGylation at Histidine Tags", BIOCONJUGATE CHEMISTRY, vol. 23, no. 2, 16 January 2012 (2012-01-16), pages 248-263, XP055074953, ISSN: 1043-1802, DOI: 10.1021/bc200530x cited in the application

## Description

This invention relates to novel conjugation reagents for conjugating polymers to proteins and peptides, and to a novel process for producing novel conjugates.

Many therapeutically active molecules, for example proteins, do not possess the properties required to achieve efficacy in clinical medical use. For example, many native proteins do not make good medicines because upon administration to patients there are several inherent drawbacks that include: (1) proteins are digested by many endo- and exo-peptidases present in blood or tissue; (2) almost all proteins are immunogenic to some extent; and (3) proteins can be rapidly excreted by kidney ultrafiltration and by endocytosis. Some molecules which might find utility as active therapeutic agents in medicines are systemically toxic or lack optimal bioavailability and pharmacokinetics. When proteins clear from the blood circulation quickly they typically have to be administered to the patient frequently. Frequent administration further increases the risk of toxicity, especially immunologically derived toxicities. Often it is difficult to achieve a therapeutically effective dose, so efficacy is compromised. Rapid clearance is therefore both an efficacy issue and a safety issue.

Water soluble, synthetic polymers, particularly polyalkylene glycols, are widely used to conjugate therapeutically active molecules such as proteins. These therapeutic conjugates have been shown to alter pharmacokinetics favourably by prolonging circulation time and decreasing clearance rates, decreasing systemic toxicity, and in several cases, displaying increased clinical efficacy. The process of covalently conjugating polyethylene glycol, PEG, to proteins is commonly known as "PEGylation".

It is important for optimised efficacy and to ensure dose to dose consistency that the number of conjugated polymer molecules per protein is the same for each molecule, and that each polymer molecule is specifically covalently conjugated to the same amino acid residue in each protein molecule. Non-specific conjugation at sites along a protein molecule results in a distribution of conjugation products and, frequently, unconjugated protein, to give a complex mixture that is difficult and expensive to purify.

WO 2005/007197 describes a series of novel conjugation reagents which can be used to react with nucleophilic groups in a protein to produce a protein-polymer conjugate. These reagents find particular utility for their ability to conjugate with both sulfur atoms derived from a disulfide bond in a protein to give thioether conjugates, and can also be used to conjugate with other nucleophiles, for example with two histidine residues, for example two histidine residues present in a polyhistidine tag attached to a protein, as described in WO 2009/047500.

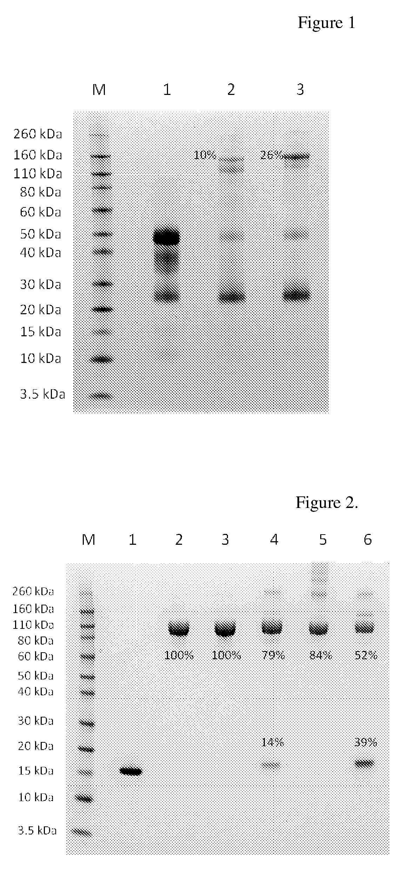

For some uses, it is desirable to conjugate two polymer chains to a protein, because the steric properties of a conjugate containing a single chain of a given molecular weight can be significantly different from the properties of a conjugate containing, for example, two chains each having half that molecular weight. Reagents capable of such conjugation are known. Thus for example US 5,932,462 (Harris) discloses reagents capable of conjugating two PEG chains to proteins. Cong et al, Bioconjugate Chemistry 23 (2012), pp. 248-263), also discloses a reagent capable of conjugating two PEG chains to proteins, specifically, the PEG-bis-sulfone reagent shown as reagent 3 of Fig. 1, p. 249. In Cong's reagent, two PEG chains are attached to different positions on a phenyl group acting as a linker to the functional protein reacting group of the reagent. Cong's reagent is capable of conjugation of two PEG chains to, for example, two sulfur atoms derived from a disulfide bond in a protein, or two histidine residues present in a polyhistidine tag attached to a protein, which provides improved conjugation compared with the reagents of Harris.

We have now found a novel reagent capable of conjugating two polymer chains to a protein, which shows improved properties over the known reagent of Cong.

Accordingly, the present invention provides a compound of the general formula: 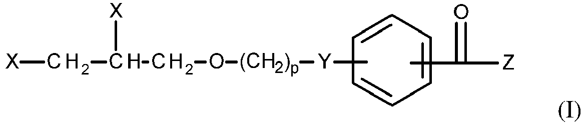 in which each X independently represents a polymer chain; p represents an integer from 1 to 6; Y represents an amide group; and Z represents either -CH.(CH₂L)₂ or-C(CH₂L)(=CH₂), in which each L independently represents a leaving group.

The reagents of the formula I contain two polymer chains X. Each polymer X may for example be a poly(alkylene glycol), a polyvinylpyrrolidone, a polyacrylate, for example poly(acryloyl morpholine), a polymethacrylate, a polyoxazoline, a polyvinylalcohol, a polyacrylamide or polymethacrylamide, for example polycarboxymethacrylamide, or a HPMA copolymer. Additionally the polymer may be one that is susceptible to enzymatic or hydrolytic degradation. Such polymers, for example, include polyesters, polyacetals, poly(ortho esters), polycarbonates, poly(imino carbonates), and polyamides, such as poly(amino acids). A polymer may be a homopolymer, random copolymer or a structurally defined copolymer such as a block copolymer. For example it may be a copolymer, e.g. a block copolymer, derived from two or more alkylene oxides, or from poly(alkylene oxide) and either a polyester, polyacetal, poly(ortho ester), or a poly(amino acid). The so-called Pluronics are an important class of PEG block copolymers. These are derived from ethylene oxide and propylene oxide blocks. Polyfunctional polymers that may be used include copolymers of divinylether-maleic anhydride and styrene-maleic anhydride.

Naturally occurring polymers may also be used, for example polysaccharides such as chitin, dextran, dextrin, chitosan, starch, cellulose, glycogen, poly(sialylic acid) and derivatives thereof. A protein may be used as the polymer. This allows conjugation of one protein, for example an antibody or antibody fragment, to a second protein, for example an enzyme or other active protein. Also, if a peptide containing a catalytic sequence is used, for example an O-glycan acceptor site for glycosyltransferase, it allows the incorporation of a substrate or a target for subsequent enzymatic reaction. Poly(amino acid)s such as polyglutamic acid or polyglycine may also be used, as may hybrid polymers derived from natural monomers such as saccharides or amino acids and synthetic monomers such as ethylene oxide or methacrylic acid.

Preferably each polymer used in the present invention is a hydrophilic or water-soluble, synthetic polymer. If a polymer is a poly(alkylene glycol), this is preferably one containing C₂ and/or C₃ units, and is especially a poly(ethylene glycol) (PEG). Except where the context requires otherwise, any reference to a polymer in this specification should be understood to include a specific reference to PEG.

A polymer may optionally be derivatised or functionalised in any desired way. Reactive groups may be linked at the polymer terminus or end group, or along the polymer chain through pendent linkers; in such cases, the polymer is for example a polyacrylamide, polymethacrylamide, polyacrylate, polymethacrylate, or a maleic anhydride copolymer. Such functionalised polymers provide a further opportunity for preparing multimeric conjugates (i.e. conjugates in which the polymer is conjugated to more than one molecule). For example, a polymer may carry one or more drug molecules at any point along its length, for example at its terminus. If desired, the polymer may be coupled to a solid support using conventional methods.

The two polymer chains X may be the same or different. Specifically, each X may represent the same chemical polymer, or a different chemical polymer. For example, each X may represent a PEG chain, or one X may represent a PEG chain and the other X may represent a different polymer, for example a PVP or a protein chain.

Each polymer X may contain a single linear chain, or it may have branched morphology composed of many chains either small or large. Generally, a polymer chain is initiated or terminated by a suitable end group, and is connected at the other end of the chain to the rest of the molecule of formula I. For example, a PEG chain may have an end group selected from alkoxy, e.g. methoxy, aryloxy, carboxy or hydroxyl. Where the chain is branched, each free branch terminus will carry the end group.

Each polymer chain X may have any suitable molecular weight, and each polymer chain X may have the same or different molecular weight as the other. For example each chain may have a molecular weight of at least 5, 10, 15, 20, 30, or 40 kDa. Generally, the preferred maximum molecular weight of each chain is 60 kDa. When a conjugate is intended to leave the circulation and penetrate tissue, for example for use in the treatment of inflammation caused by malignancy, infection or autoimmune disease, or by trauma, it may be advantageous to use a conjugate in which the total molecular weight of the polymers (X+X) is in the range 2000-30,000g/mol. For applications where the conjugate is intended to remain in circulation it may be advantageous to use a higher total molecular weight of polymer, for example in the range of 20,000 - 75,000g/mol.

p represents an integer from 1 to 6, for example 1, 2 or, especially, 3.

The reagents of the present invention contain an amide group, Y, which as drawn in the formula I may be -CO-NR'- or, preferably, -NR'-CO-, in which R' represents a C₁₋₄alkyl group, for example a methyl group, or, especially, a hydrogen atom. This group may be linked to the phenyl group shown in formula I at any position, but is preferably in the para position relative to the -CO.Z group. The phenyl group of the formula I may carry additional substituents if desired, but is preferably unsubstituted.

A leaving group L may for example represent -SR, -SO₂R, -OSO₂R,-N⁺R₃,-N⁺HR₂,-N⁺H₂R, halogen, or -O∅, in which R has the meaning given above, and ∅ represents a substituted aryl, especially phenyl, group, containing at least one electron withdrawing substituent, for example -CN,-NO₂, -CO₂R, -COH, -CH₂OH, -COR, -OR, -OCOR, -OCO₂R, -SR,-SOR, -SO₂R, -NHCOR, -NRCOR, -NHCO₂R, -NRCO₂R, -NO, -NHOH, -NROH, -C=N-NHCOR, -C=N-NRCOR, -N⁺R₃, -N⁺HR₂, -N⁺H₂R, halogen, for example chlorine or, especially, bromine or iodine, -C≡CR, -C=CR₂ and -C=CHR, in which each R independently has one of the meanings given above. Alkyl or aryl sulfonyl groups are particularly preferred leaving groups, with phenylsulfonyl or, especially, tosyl, being especially preferred. Where two Ls are present, these may be different groups, but preferably they are the same group.

Except where otherwise stated, substituents which may be present on any optionally substituted aryl, for example phenyl, or heteroaryl group present in a compound of formula I include for example one or more of the same or different substituents selected from alkyl (preferably C₁₋₄alkyl, especially methyl, optionally substituted by OH or CO₂H), -CN, -NO₂, -CO₂R, -COH, -CH₂OH, -COR, -OR, -OCOR, -OCO₂R, -SR, -SOR, -SO₂R, - NHCOR, -NRCOR, NHCO₂R, -NR.CO₂R, -NO, -NHOH, -NR.OH, -C=N-NHCOR, -C=N-NR.COR, -N⁺R₃, -N⁺H₃, -N⁺HR₂, -N⁺H₂R, halogen, for example fluorine or chlorine, -C≡CR, -C=CR₂ and -C=CHR, in which each R independently has one of the meanings given above. Preferred substituents, if present, include for example CN, NO₂, -OR, -OCOR, -SR, -NHCOR, -NR.COR, -NHOH and -NR.COR.

Especially preferred reagents according to the invention have the formulae: 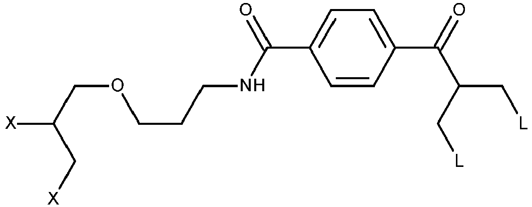 or

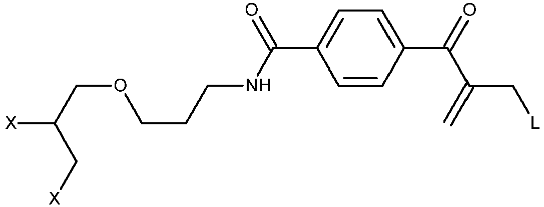

In these reagents, preferably X is polyethylene glycol, especially methoxy-terminated polyethylene glycol, i.e. CH₃O-(CH₂CH₂O)ₘ- in which m is the number of ethylene oxide units in the PEG. In addition, in these reagents, preferably each L is a tosyl group, thus: 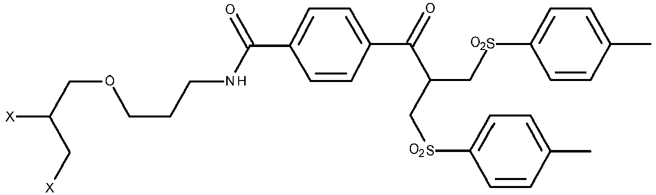 or

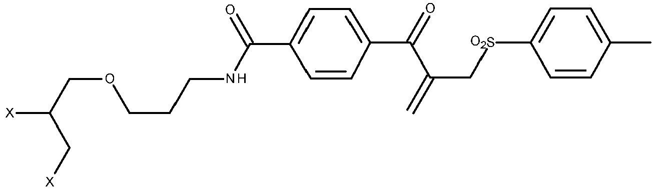

The compounds of formula I may be used for conjugation to a protein or peptide. For convenience, the term "protein" will be used throughout this Specification, and except where the context requires otherwise, the use of the term "protein" should be understood to include a reference to peptide.

Accordingly, the invention further provides a process for the preparation of a polymer conjugate, which comprises reacting a compound of the general formula I with a protein or a peptide. The resulting conjugates have the general formula: 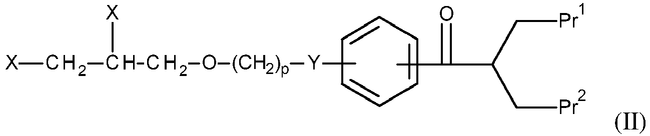 in which X, p and Y have the meanings given above, and either each of Pr¹ and Pr² represents a separate protein or peptide molecule, or Pr¹ and Pr² together represent a single protein or peptide Pr bonded at two separate points, thus:

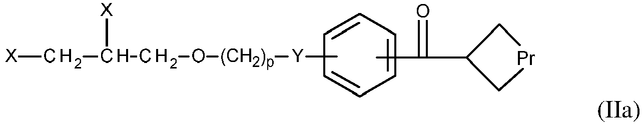

Preferably Pr¹ and Pr² together represent a single protein bonded to two sulfur atoms derived from a disulfide bond in a protein, or to two histidine residues present in a polyhistidine tag attached to a protein (i.e. the resulting conjugates have the general formula IV(a)).

In the reagent of formula I, Z represents either -CH.(CH₂L)₂ or -C(CH₂L)(=CH₂). These two groups are chemically equivalent to each other. If a reagent of formula I in which Z represents -CH.(CH₂L)₂, i.e. a reagent of formula Ia: 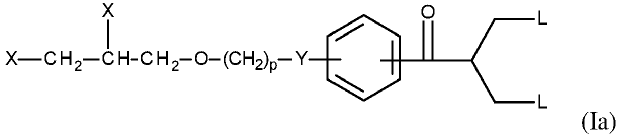 is used to react with a protein in a process according to the invention, the reaction proceeds by the loss of one leaving group L, and resultant formation of a reagent of formula I in which Z represents -C(CH₂L)(=CH₂), i.e. a reagent of formula Ib:

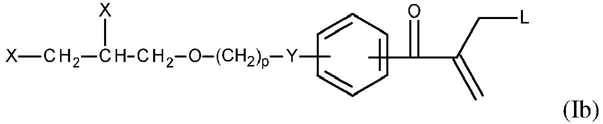

This reagent reacts with one nucleophile, for example a cysteine, histidine or lysine residue, in the protein. Subsequently, the remaining leaving group L is lost, and reaction with a second nucleophile (either in a second molecule of protein or in the same protein molecule as the first nucleophile) occurs to form the desired conjugate. Therefore, the process of the invention can be carried out using a compound of formula Ia as a starting material, in which case a compound of formula Ib is formed *in situ,* or a pre-formed compound of formula Ib may be used as starting material.

The conjugation reaction according to the invention may be carried out under the reaction conditions described in WO 2005/007197 and WO 2009/047500. The process may for example be carried out in a solvent or solvent mixture in which all reactants are soluble. For example, the protein may be allowed to react directly with the polymer conjugation reagent in an aqueous reaction medium. This reaction medium may also be buffered, depending on the pH requirements of the nucleophile. The optimum pH for the reaction will generally be at least 4.5, typically between about 5.0 and about 8.5, preferably about 6.0 to 7.5. The optimal reaction conditions will of course depend upon the specific reactants employed.

Reaction temperatures between 3-37°C are generally suitable when using an aqueous reaction medium. Reactions conducted in organic media (for example THF, ethyl acetate, acetone) are typically conducted at temperatures up to ambient.

Where bonding to the protein is via two sulfur atoms derived from a disulfide bond in the protein, the process may be carried out by reducing the disulfide bond *in situ* following which the reduced product reacts with the reagent of the formula I. Preferably the disulfide bond is reduced and any excess reducing agent is removed, for example by buffer exchange, before the conjugation reagent is introduced. The disulfide can be reduced, for example, with dithiothreitol, mercaptoethanol, or tris-carboxyethylphosphine using conventional methods.

The protein can be effectively conjugated using a stoichiometric equivalent or a slight excess of conjugation reagent I. However, it is also possible to conduct the conjugation reaction with an excess stoichiometry of conjugation reagent, and this may be desirable for some proteins. The excess reagent can easily be removed, for example by ion exchange chromatography, during subsequent purification of the conjugate.

Compounds of the general formula I in which Z represents -CH.(CH₂L)₂ may be prepared by either reacting a compound of the general formula I 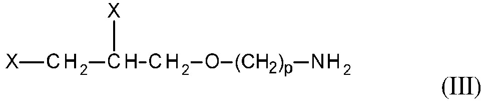 with a compound of the general formula 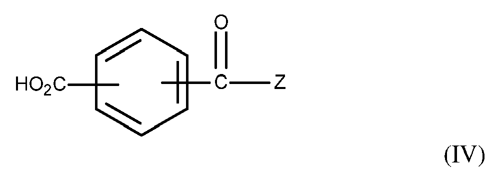 or by reacting a compound of the general formula 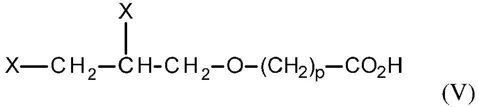 with a compound of the general formula

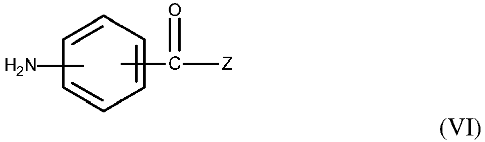

In both cases, an amide group is formed. As is well known in the art, the CO₂H group which is reacted to form the amide group, is suitably activated to facilitate the reaction, for example by formation of an active ester, an acyl chloride, or an anhydride, or directly with amine by the use of an activation agent such as a carbodiimide.

As explained above, compounds of the general formula I in which Z represents -C(CH₂L)(=CH₂) may be prepared by removing a leaving group L from the corresponding compound of the general formula I in which Z represents a -CH.(CH₂L)₂ group.

The immediate product of the process according to the invention is a conjugate which still contains the keto group CO attached to the phenyl ring in formula I, i.e. a conjugate of formula II, especially IIa, above. However, the process of the invention is reversible under suitable conditions. This may be desirable for some applications, for example where rapid release of the protein is required, but for other applications, rapid release of the protein may be undesirable. It may therefore be desirable to stabilise the conjugates by reduction of the keto group to give a moiety which prevents release of the protein, typically a hydroxyl group OH, although reductive amination is also a possibility, giving an amine group CH.NH₂, CH.NHR or CH.NR₂ in which each R independently has the meaning given above. These groups may be further reacted if desired, for example a hydroxy group may be converted into an ether group CH.OR by reaction with an etherifying agent; an ester group CH.O.C(O)R may be obtained by the reaction of a hydroxy group with an acylating agent; or an amide CH.NHC(O)R or CH.N(C(O)R)₂ may be formed by acylation of an amine. Accordingly, the process according to the invention may comprise the additional step of reducing the keto group in the conjugate. The use of a borohydride, for example sodium borohydride, sodium cyanoborohydride, potassium borohydride or sodium triacetoxyborohydride, as reducing agent is particularly preferred. Other reducing agents which may be used include for example tin(II) chloride, alkoxides such as aluminium alkoxide, and lithium aluminium hydride.

Conjugates preparable by the process of the present invention are novel, and therefore form part of the present invention *per se.* Novel conjugates according to the present invention have the general formula: 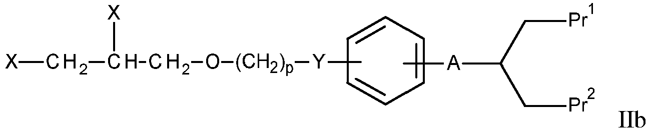 in which X, p and Y have the meanings given above, A represent a group CO, CHOH, CH.NH₂, CH.NHR, CH.NR₂, CH.OR CH.O.C(O)R, CH.NHC(O)R, or CH.N(C(O)R)₂, in which each R has the meaning given above, and either each of Pr¹ and Pr² represents a separate protein or peptide molecule or both of Pr¹ and Pr² together represent a single protein or peptide bonded at two separate points. Preferred conjugates have the general formula: 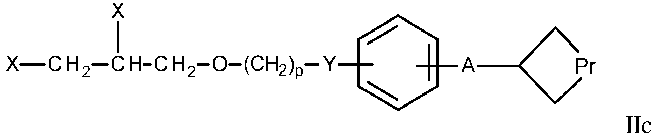 in which X, p, Y and A have the meanings given above, and Pr represents a single protein or peptide bonded at two separate points.

Of course, it is possible for more than one conjugating reagent of the formula I to be conjugated to a protein, where the protein contains sufficient suitable attachment points. For example, in a protein which contains two different disulfide bonds, or in a protein which contains one disulfide bond and also carries a polyhistidine tag, it is possible to conjugate two molecules of the reagent of formula I per molecule of protein.

Suitable proteins which may be conjugated using the process of the invention include for example peptides, polypeptides, antibodies, antibody fragments, enzymes, cytokines, chemokines, receptors, blood factors, peptide hormones, toxin, transcription proteins, or multimeric proteins.

The following gives some specific proteins which may be conjugated using the present invention. Enzymes include carbohydrate-specific enzymes, proteolytic enzymes and the like, for example the oxidoreductases, transferases, hydrolases, lyases, isomerases and ligases disclosed by US 4,179,337. Specific enzymes of interest include asparaginase, arginase, adenosine deaminase, superoxide dismutase, catalase, chymotrypsin, lipase, uricase, bilirubin oxidase, glucose oxidase, glucuronidase, galactosidase, glucocerbrosidase, glucuronidase, and glutaminase.

Blood proteins include albumin, transferrin, Factor VII, Factor VIII or Factor IX, von Willebrand factor, insulin, ACTH, glucagen, somatostatin, somatotropins, thymosin, parathyroid hormone, pigmentary hormones, somatomedins, erythropoietin, luteinizing hormone, hypothalamic releasing factors, antidiuretic hormones, prolactin, interleukins, interferons, for example IFN-α or IFN-β, colony stimulating factors, hemoglobin, cytokines, antibodies, antibody fragments, chorionicgonadotropin, follicle-stimulating hormone, thyroid stimulating hormone and tissue plasminogen activator.

Other proteins of interest are allergen proteins disclosed by Dreborg et al Crit. Rev. Therap. Drug Carrier Syst. (1990) 6 315-365 as having reduced allergenicity when conjugated with a polymer such as poly(alkylene oxide) and consequently are suitable for use as tolerance inducers. Among the allergens disclosed are Ragweed antigen E, honeybee venom, mite allergen and the like.

Glycopolypeptides such as immunoglobulins, ovalbumin, lipase, glucocerebrosidase, lectins, tissue plasminogen activator and glycosilated interleukins, interferons and colony stimulating factors are of interest, as are immunoglobulins such as IgG, IgE, IgM, IgA, IgD and fragments thereof.

Of particular interest are receptor and ligand binding proteins and antibodies and antibody fragments which are used in clinical medicine for diagnostic and therapeutic purposes. The antibody may be used alone or may be covalently conjugated ("loaded") with another atom or molecule such as a radioisotope or a cytotoxic/antiinfective drug. Epitopes may be used for vaccination to produce an immunogenic polymer - protein conjugate.

Particularly preferred proteins include antibody fragments, for example IgG Fab fragment, and interferons, such as IFN-α, IFN-β and consensus IFN.

The protein may be derivatised or functionalised if desired. In particular, prior to conjugation, the protein, for example a native protein, may have been reacted with various blocking groups to protect sensitive groups thereon; or it may have been previously conjugated with one or more polymers or other molecules, either using the process of this invention or using an alternative process. In one preferred embodiment of the invention, it contains a polyhistidine tag, which can be targeted by the conjugation reagent according to the invention.

The invention further provides a pharmaceutical composition comprising a conjugate according to the invention together with a pharmaceutically acceptable carrier, and optionally also containing a further active ingredient in addition to the conjugate according to the invention; a conjugate according to the invention for use in therapy; and the use of a conjugate according to the invention in a process for the manufacture of a medicament. The invention finds utility in a method of treating a patient which comprises administering a pharmaceutically-effective amount of a conjugate or a pharmaceutical composition according to the invention to a patient.

The conjugating reagents of the present invention have been found to be extremely useful, being capable of highly efficient site-specific conjugation to proteins, the resulting novel conjugates demonstrating a high level of stability. As illustrated in the examples below, dramatically improved efficiency over the comparable known reagent of Cong et al, Bioconjugate Chemistry 23 (2012), pp. 248-263, is obtained.

The accompanying drawings illustrate results obtained in the following Examples:
Figure 1 illustrates the SDS-PAGE gel obtained in Example 2.
Figures 2, 3 and 4 illustrate the SDS-PAGE gels obtained in Example 3.
Figure 5 illustrates the SDS-PAGE gel obtained in Example 4.

The following Examples illustrate the invention.

### Example 1: Preparation of PEG reagent 1

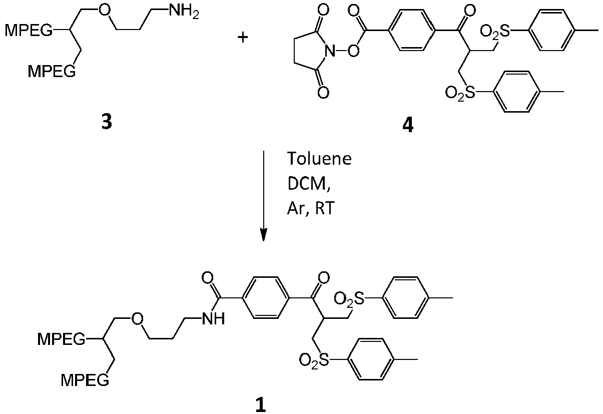

40 (2x20) kDa bifurcated PEG amine **3** (MPEG being CH₃O.CH₂CH₂O)ₘ-) was purchased from NOF CORPORATION (SUNBRIGHT GL2-400 PA, lot: M7D902). 4-[2,2-*bis*[(*p-*tolylsulfonyl)methyl] acetyl]benzoic acid-NHS ester, **4** was prepared according to Brocchini et al. Nat. Protoc. 2006, 1(4), 2241-2252.

To a single neck round-bottomed flask containing a magnetic stirrer bar, was added bifurcated PEG amine **3** (300 mg) and toluene (8 mL). The resulting homogeneous solution was evaporated under reduced pressure using a rotary evaporator for 2 h to leave a solid residue. The residue was dissolved in dichloromethane (15 mL), the flask was sealed with a septum and the mixture stirred under argon. To the solution was added activated linker **4** (27 mg), the flask was resealed with a septum and the reaction was stirred at rt overnight. The septum was removed and the volatile portion was removed via evaporation under reduced pressure using a rotary evaporator. Acetone (20 mL) was added to the residue and the solid was dissolved with gentle warming (30 °C). The resulting solution was filtered through non-absorbent cotton wool into a 50 mL Falcon tube. Cooling the solution in a dry-ice bath resulted in a thick precipitate. Centrifugation (-9 °C, 4000 rpm) for 30 min sedimented the precipitate. The supernatant was decanted and the pellet was again dissolved in acetone (20 mL) at 30 °C. Precipitation, sedimentation and decanting were performed as previously described. A third cycle of acetone precipitation and sedimentation was performed and after decanting the supernatant, the pellet was frozen at -80 °C and then dried to constant mass under high vacuum to give PEG reagent **1** as an off-white solid (227 mg). ¹H NMR (CDCl₃): δ (ppm) 2.49 (s, 6H), 3.38 (s, 6H), 3.45-3.86 (m), 4.33 (m, 1H), 7.36 (AA'BB', 4H), 7.64 (AA'BB', 2H), 7.68 (AA'BB', 4H), 7.83 (AA'BB', 2H).

### Example 2: Comparison of the reactivity of PEG reagents 1 and 2 with a Human IgG, Fab Fragment

In this example, PEG reagent 1 of Example 1 was compared with the following reagent, PEG reagent 2, in which MPEG is CH₃O.(CH₂CH₂O)ₘ₋₁-CH₂CH₂-, of Cong *et al,* supra.:

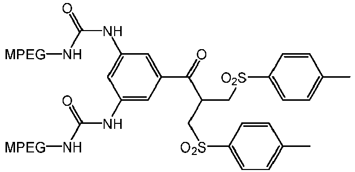

A human IgG, Fab fragment solution (4.0 mg, 0.909 mL) Jackson ImmunoResearch Laboratories Inc. Cat. No. 009-000-007) was diluted to 4.95 mL with 50 mM sodium phosphate, pH 7.4 (containing 150 mM NaCl and 20 mM EDTA). To the Fab fragment solution, 1.0 M DTT (50 µL) was added to give a final DTT concentration of 10 mM in order to reduce the interchain disulfide bond so that PEGylation could occur. The resulting solution was mixed gently and then stood at 4 °C for 1 h. The solution of reduced Fab was buffer exchanged into 50 mM sodium phosphate, pH 7.4 (containing 150 mM NaCl and 20 mM EDTA) using PD-10 desalting columns. The reduced Fab solution was split equally into two portions (3.5 mL, ~2 mg). Two PEG reagents: PEG reagent **1** and PEG reagent **2,** were dissolved in 50 mM sodium phosphate, pH 7.4 (containing 150 mM NaCl and 20 mM EDTA) at 20 mg/mL. To the first portion of Fab solution, PEG reagent **1** (75 µL, 1.5 mg) was added and to the second portion of Fab solution, PEG reagent **2** (75 µL, 1.5 mg) was added. Both reactions were mixed gently and then stood at 4 °C for 20 h. After 20 h the crude reaction mixtures were analysed by SDS-PAGE. The gel was stained with InstantBlue™ and imaged using an IMAGEQUANT™ LAS 4010 instrument. The result is shown in Figure 1. In Figure 1, lane M indicates Novex Protein Standards; lane 1 indicates human IgG, Fab fragment; lane 2 indicates PEGylated product from PEG reagent **2** at 20 h; and lane 3 indicates PEGylated product from PEG reagent **1** at 20 h. From the SDS-PAGE analysis it can be seen that, while both PEG reagents **1** and **2** were successfully conjugated to the Fab fragment, the efficiency of the conjugation for PEG reagent **1** was 26%, over double of that for PEG reagent **2** (10%).

### Example 3: Stability comparisons of IFN α-2a conjugates prepared with PEG reagents 1 and 2.

*Preparation of conjugates:* A solution of IFN α-2a (6.5 mg, 0.845 mg/mL) was prepared in 50 mM sodium phosphate buffer, pH 7.4 (containing 150 mM NaCl and 20 mM EDTA). The protein solution was diluted with buffer (313 µL) and a 1.0 mM DTT solution in water (187.5 mL) was then added to give a final DTT concentration of 25 mM and a reaction volume of 7.5 mL. After gentle mixing, the reaction was stood at room temperature for 30 min. The reduced protein was buffer exchanged into 50 mM sodium phosphate, pH 7.4 (containing 150 mM NaCl and 20 mM EDTA) using PD-10 columns. The eluted protein solution was centrifuged (3000 g, 4 °C, 5 min) and the supernatant was then quantified by UV absorbance measurements at 280 nm (0.532 mg/mL). The protein solution was diluted to 0.10 mg/mL with buffer. PEGs **1** and **2** were dissolved at 20 mg/mL in 50 mM sodium phosphate, pH 7.4 (containing 150 mM NaCl and 20 mM EDTA). Two vials were each charged with reduced IFN α-2a (2.5 mg, 24.8 mL); to the first vial PEG reagent **1** (4.9 mg, 0.245 mL) was added and to the second vial PEG reagent **2** (4.9 mg, 0.245 mL) was added. The reactions were mixed gently and then stood at 4 °C for 18 h. Any reduced protein in the final reaction mixtures was oxidised by sequentially adding 5 mg/mL copper sulfate (12.18 µL) and then 50:50 (mM) GSH/GSSG (0.25 mL). The reoxidation reaction was conducted at 4 °C overnight. The reaction mixtures were diluted x4 with 100 mM sodium acetate, pH 4 and then purified by cation exchange chromatography (Macrocap™ SP) using a step gradient elution of 100 mM sodium acetate, pH 4 (1.0 M NaCl) with the desired conjugates eluting at 0.60-0.65 M NaCl.

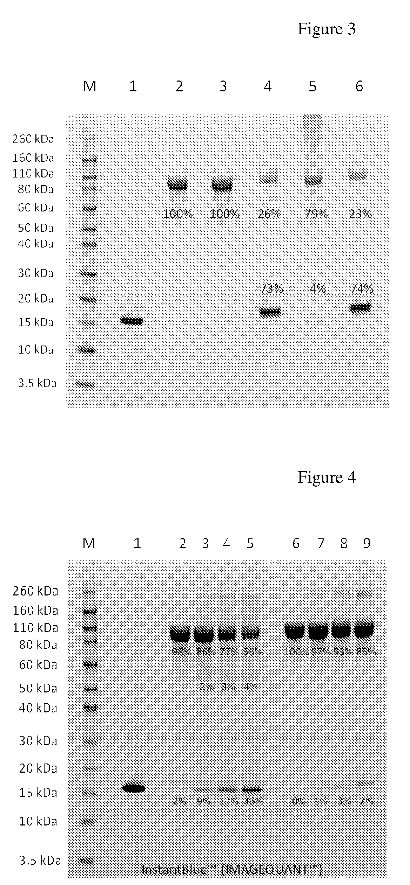

*Stability comparison 1. Stress Tests for IFN* α-2a *conjugates prepared with PEG reagents **1** and 2:* For each of the IFN α-2a samples PEGylated with **1** and **2** (in filter sterilised PBS), four vials were prepared. Each vial was loaded with 20 µL of conjugate at a concentration of 200 µg/mL. Two vials for each of the test samples contained 10 mM DTT. One vial with and one vial without DTT were heated to 50 °C for 1 h. The remaining vials were heated to 90 °C for 10 min. The samples (along with unconjugated protein and unstressed conjugate) were analysed by SDS-PAGE - the gels were stained with InstantBlue™ and imaged using an IMAGEQUANT™ LAS 4010 instrument and the results are shown in Figures 2 and 3 for PEG 1-IFN α-2a and PEG 2-IFN α-2a respectively. In Figures 2 and 3, lane M indicates Novex Protein Standards; lane 1 indicates IFN α-2a; lane 2 indicates PEG-IFN α-2a; lane 3 indicates PEG-IFN α-2a - 50 °C, 1 h; lane 4 indicates PEG-IFN α-2a - 50 °C, DTT, 1 h; lane 5 indicates PEG-IFN α-2a - 90 °C, DTT, 10 min; and lane 6 indicates PEG-IFN α-2a - 90 °C, DTT, 10 min.

Figures 2 and 3 show that both of the conjugates tested were stable at 50 °C for 1 h. However, after heating at 50 °C for 1 h in the presence of 10 mM DTT, significantly more free protein and aggregation is observed for the conjugate prepared with PEG reagent **2.** Thermal stress at 90 °C for 10 min resulted in release of free protein for the conjugate PEGylated with **2** but not for the conjugate PEGylated with **1.**

*Stability comparison* 2. *28 Day, 40 °C, accelerates stability studies for IFN α-2a conjugates PEGylated with PEG reagents **1** and **2.*** Solutions of the two test samples were made up in filter sterilised PBS (containing 0.01 % (w/v) NaN₃) at a protein concentration of 200 µg/mL. For each of PEG **1**-IFN α-2a and PEG **2**-IFN α-2a four vials were loaded with 100 µL of test sample. One vial for each sample was immediately frozen at -80 °C (t = 0 days). The remaining three were sealed with parafilm® and then were stored at 40 °C. At 7, 14 and 28 days a sample was removed from storage and frozen at -80 °C until the completion of the study. The test samples were flash thawed in a water bath thermostated at 37 °C and analysed by SDS-PAGE (InstantBlue™ stained and imaged using an IMAGEQUANT™ LAS 4010 instrument) and the result is shown in Figure 4, in which lane M indicates Novex Protein Standards; lane 1 indicates IFN α-2a (1 µg); lane 2 indicates PEG **2**-IFN α-2a, Day 0; lane 3 indicates PEG **2**-IFN α-2a, Day 7; lane 4 indicates PEG **2**-IFN α-2a, Day 14; lane 5 indicates PEG **2**-IFN α-2a, day 28; lane 6 indicates PEG **1-**IFN α-2a, Day 0; lane 7 indicates PEG **1**-IFN α-2a, Day 7; lane 8 indicates PEG **1**-IFN α-2a, Day 14; lane 6 indicates PEG **1**-IFN α-2a, Day 28. In Figure 4, it can clearly be seen that the PEG **2**-IFN α-2a is less stable than PEG **1**-IFN α-2a with more free protein and less conjugate remaining at each time point.

### Example 4: Conjugation of IFN-β-1b with PEG reagent 1.

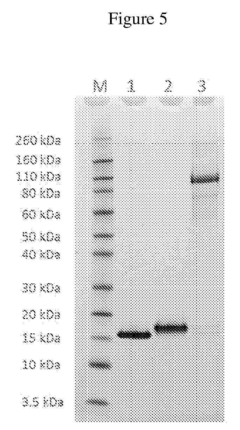

To disulfide reduced IFN-β-1b (9.5 mg, 0.3 mg/mL) at pH 7.3 was added a solution of PEG reagent 1 (1.7 mL, 20 mg/mL) at pH 7.3. The resulting solution was allowed to incubate at 22 °C for 4 h, whereupon the crude reaction mixture was analysed by SDS-PAGE. The gel was stained with InstantBlue™ and imaged using an IMAGEQUANT™ LAS 4010 instrument. The result is shown in Figure 5. In Figure 5, in lane M are Novex Protein Standards; lane 1 is the starting IFN-β-1b; lane 2 is the reduced IFN-β-1b and lane 3 is the reaction mixture of PEG reagent **1** with IFN-β-1b. From the SDS-PAGE analysis it can be seen that PEGylation of IFN-β-1b occurred successfully with a product band visible level with the 110 kDa protein standard.

## Claims

1. A compound of the general formula: 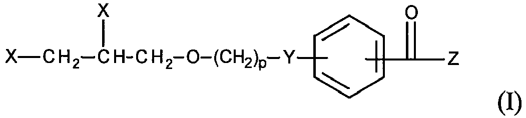 in which each X independently represents a polymer chain; p represents an integer from 1 to 6; Y represents an amide group; and Z represents either -CH.(CH₂L)₂ or -C(CH₂L)(=CH₂), in which each L independently represents a leaving group.

2. A compound as claimed in claim 1, in which each X represents a poly(alkylene glycol), polyvinylpyrrolidone, polyacrylate, polymethacrylate, polyoxazoline, polyvinylalcohol, polyacrylamide, polymethacrylamide, HPMA copolymer, polyester, polyacetal, poly(ortho ester), polycarbonate, poly(imino carbonate), polyamide, or polysaccharide.

3. A compound as claimed in claim 1, in which each X represent poly(ethylene glycol).

4. A compound as claimed in any one of the preceding claims, in which Y represents -NH-CO-.

5. A compound as claimed in any one of the preceding claims, in which p is 3.

6. A compound as claimed in any one of the preceding claims, in which each L independently represents -SR, -SO₂R, -OSO₂R,-N⁺R₃,-N⁺HR₂,-N⁺H₂R, halogen, or -O∅, in which R represents a hydrogen atom or an alkyl, aryl or alkyl-aryl group, and ∅ represents a substituted aryl group containing at least one electron withdrawing substituent.

7. A compound as claimed in claim 6, in which each L independently represents phenylsulfonyl or tosyl.

8. A compound as claimed in claim 1, having the formula: 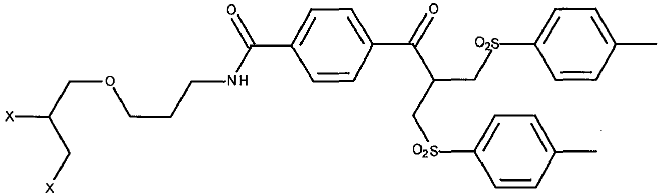 or

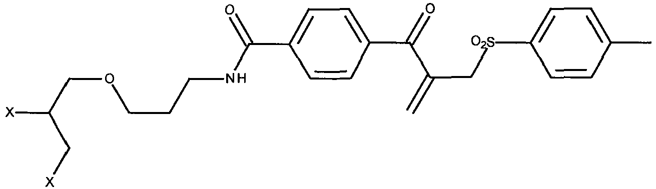

9. A compound as claimed in claim 8, in which each X independently represents polyethylene glycol of the formula CH₃O-(CH₂CH₂O)ₘ- in which m is the number of ethylene oxide units in X.

10. A compound of the general formula: 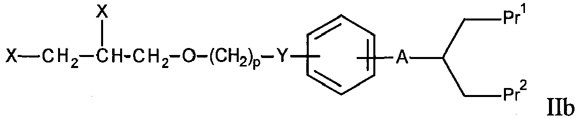 in which X, p and Y have the meanings given in any one of the preceding claims, A represent a group CO, CHOH, CH.NH₂, CH.NHR, CH.NR₂, CH.OR CH.O.C(O)R, CH.NHC(O)R, or CH.N(C(O)R)₂, in which each R represents a hydrogen atom or an alkyl, aryl or alkyl-aryl group, and either each of Pr¹ and Pr² represents a separate protein or peptide molecule or both of Pr¹ and Pr² together represent a single protein or peptide bonded at two separate points.

11. A compound as claimed in claim 10, having the general formula: 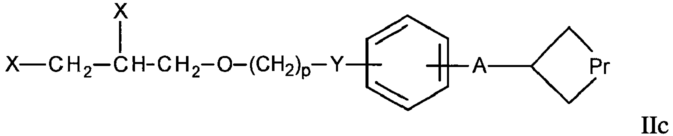 in which X, p, Y and A have the meanings given in claim 10, and Pr represents a single protein or peptide bonded at two separate points.

12. A compound as claimed in claim 11, in which Pr represents a single protein or peptide bonded to two sulfur atoms derived from a disulfide bond in said protein or peptide, or to two histidine residues present in a polyhistidine tag attached to said protein or peptide.

13. A compound as claimed in either claim 11 or claim 12, in which Pr represents an IgG Fab fragment, INF-α, IFN-β, or consensus IFN.

14. A process for the preparation of a compound as claimed in any one of claims 10 to 13, which comprises reacting a compound as claimed in any one of claims 1 to 9 with a protein or a peptide; and optionally reducing the keto group in the resulting conjugate.

15. A pharmaceutical composition comprising a compound as claimed in any one of claims 1 to 9, together with a pharmaceutically acceptable carrier, and optionally also containing a further active ingredient.

16. A compound as claimed in any one of claims 1 to 9 or a composition as claimed in claim 15, for use in therapy.

## Patentansprüche

1. Verbindung der allgemeinen Formel: 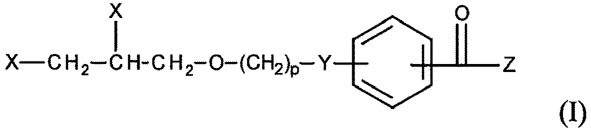 in der jedes X unabhängig eine Polymerkette darstellt; p eine ganze Zahl von 1 bis 6 darstellt; Y eine Amidgruppe darstellt; und Z entweder -CH.(CH₂L) ₂ oder -C (CH₂L) (=CH₂) darstellt, in denen L jeweils unabhängig eine Abgangsgruppe darstellt.

2. Eine Verbindung, wie in Anspruch 1 beansprucht, in der jedes X ein Poly(alkylenglycol), ein Polyvinylpyrrolidon, ein Polyacrylat, ein Polymethacrylat, ein Polyoxazolin, einen Polyvinylalkohol, ein Polyacrylamid, ein Polymethacrylamid, ein HPMA - Copolymer, einen Polyester, ein Polyacetal, einen Poly(orthoester), ein Polycarbonat, ein Poly(imincarbonat), ein Polyamid oder ein Polysaccharid darstellt.

3. Eine Verbindung, wie in Anspruch 1 beansprucht, in der jedes X ein Poly(ethylenglycol) darstellt.

4. Eine Verbindung, wie in einem der vorhergehenden Ansprüche beansprucht, in der Y die Gruppe -NH-CO- darstellt.

5. Eine Verbindung, wie in einem der vorhergehenden Ansprüche beansprucht, in der p die Zahl 3 ist.

6. Eine Verbindung, wie in einem der vorhergehenden Ansprüche beansprucht, in der jedes L unabhängig voneinander -SR, -SO₂R, -OSO₂R, -N⁺R₃, -N⁺HR₂, -N⁺H₂R, Halogen, oder -00 darstellt, wobei in diesen R ein Wasserstoffatom oder eine Alkyl-, Aryl- oder Alkylaryl- Gruppe darstellt, und 0 eine substituierte Arylgruppe, welche mindestens einen elektronenziehenden Substituenten enthält, darstellt.

7. Eine Verbindung, wie in Anspruch 6 beansprucht, in der jedes L unabhängig Phenylsulfonyl oder Tosyl darstellt.

8. Eine Verbindung, wie in Anspruch 1 beansprucht, welche die Formel: 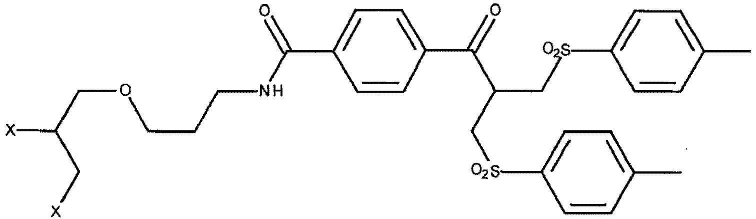 oder 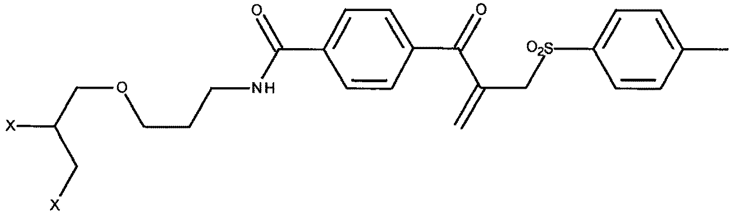 aufweist.

9. Eine Verbindung, wie in Anspruch 8 beansprucht, in der jedes X unabhängig voneinander ein Polyethylenglycol der Formel CH₃0-(CH₂CH₂O)ₘ- darstellt, wobei in dieser m die Anzahl der Ethylenoxideinheiten in X angibt.

10. Eine Verbindung der allgemeinen Formel: 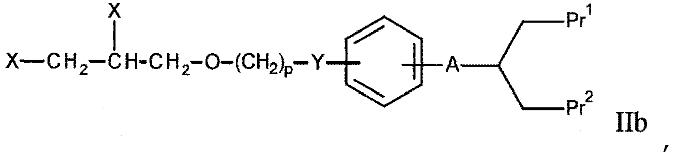 in der X, p und Y die Bedeutungen haben, welche in einem der vorhergehenden Ansprüche angegebenen sind, A eine Gruppe CO, CHOH, CH.NH₂, CH.NHR, CH.NR₂, CH.OR CH.O.C(O)R, CH.NHC(O)R, oder CH.N(C(O)R)₂ darstellt, wobei in diesen jedes R ein Wasserstoffatom oder eine Alkyl-, eine Aryl- oder eine Alkylaryl- Gruppe darstellt, und entweder Pr¹ und Pr² einzeln jeweils ein Protein oder Peptidmolekül darstellen oder Pr¹ und Pr² beide zusammen ein einzelnes Protein oder Peptid darstellen, welches an zwei getrennten Punkten gebunden ist.

11. Eine Verbindung, wie in Anspruch 10 beansprucht, welche die allgemeine Formel aufweist: 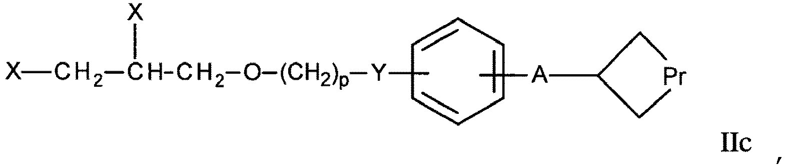 in der X, p, Y und A die in Anspruch 10 angegebenen Bedeutungen haben, und Pr ein einzelnes Protein oder Peptid darstellt, welches an zwei getrennten Punkten gebunden ist.

12. Eine Verbindung, wie in Anspruch 11 beansprucht, in der Pr ein einzelnes Protein oder Peptid darstellt, welches an zwei Schwefelatome, die aus einer Disulfidbindung in diesem Protein oder Peptid hervorgegangen sind, oder an zwei Histidinreste, welche in einem Polyhistidin-Tag, der an dieses Protein oder Peptid angebrachtr ist, vorhanden sind, gebunden ist.

13. Eine Verbindung, wie in entweder in Anspruch 11 oder Anspruch 12 beansprucht, in der Pr ein IgG, ein Fab-Fragment, ein INF-α, ein IFN-β oder ein Konsensus-IFN darstellt.

14. Ein Verfahren zur Herstellung einer Verbindung, wie in einem der Ansprüche 10 bis 13 beansprucht, welches umfasst: das Umsetzen einer Verbindung, wie in einem der Ansprüche 1 bis 9 beansprucht, mit einem Protein oder einem Peptid; und gegebenenfalls das Reduzieren der Ketogruppe in dem erhaltenen Konjugat.

15. Pharmazeutische Zusammensetzung, umfassend eine Verbindung, wie in einem der Ansprüche 1 bis 9 beansprucht, zusammen mit einem pharmazeutisch akzeptablen Träger, und gegebenenfalls auch einen weiteren Wirkstoff enthaltend.

16. Eine Verbindung, wie in einem der Ansprüche 1 bis 9 beansprucht, oder eine Zusammensetzung, wie in Anspruch 15 beansprucht, zur Verwendung bei einer Therapie.

## Revendications

1. Composé de formule générale : 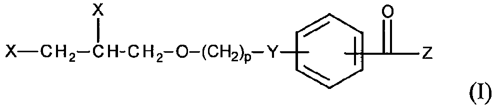 dans laquelle chaque groupe X représente indépendamment un chaîne polymère ; p représente un nombre entier de 1 à 6 ; Y représente un groupe amide ; et Z représente un groupe -CH.(CH₂L)₂ ou -C(CH₂L) (=CH₂), dans lequel chaque groupe L représente indépendamment un groupe partant.

2. Composé suivant la revendication 1, dans lequel chaque groupe X représente un poly(alkylèneglycol), la polyvinylpyrrolidone, un polyacrylate, un polyméthacrylate, une polyoxazoline, un poly(alcool vinylique), un polyacrylamide, un polyméthacrylamide, un copolymère HPMA, un polyester, un polyacétal, un poly(orthoester), un polycarbonate, un poly(iminocarbonate), un polyamide ou un polysaccharide.

3. Composé suivant la revendication 1, dans lequel chaque groupe X représente un poly(éthylèneglycol).

4. Composé suivant l'une quelconque des revendications précédentes, dans lequel Y représente un groupe -NH-CO-.

5. Composé suivant l'une quelconque des revendications précédentes, dans lequel p est égal à 3.

6. Composé suivant l'une quelconque des revendications précédentes, dans lequel chaque groupe L représente indépendamment un groupe -SR, -SO₂R, -OSO₂R, -N⁺R₃, -N⁺HR₂, -N⁺H₂R, halogéno ou -O∅, dans lequel R représente un atome d'hydrogène ou un groupe alkyle, aryle ou alkylaryle, et ∅ représente un groupe aryle substitué contenant au moins un substituant électrophile.

7. Composé suivant la revendication 6, dans lequel chaque groupe L représente indépendamment un groupe phényl-sulfonyle ou tosyle.

8. Composé suivant la revendication 1, répondant à la formule 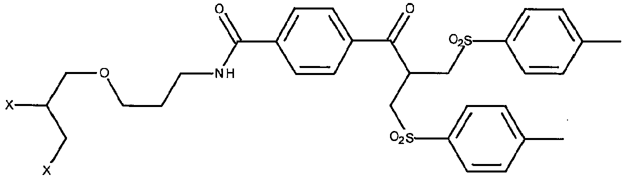 ou

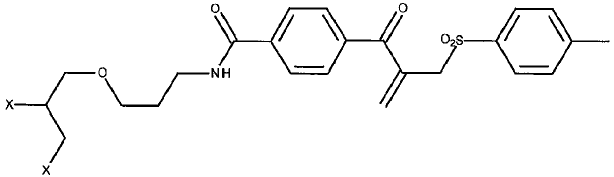

9. Composé suivant l'une quelconque des revendications précédentes, dans lequel chaque groupe X représente indépendamment un polyéthylèneglycol de formule CH₃O-(CH₂CH_{2O})ₘ- dans laquelle m représente le nombre de motifs oxyde d'éthylène dans X.

10. Composé de formule générale : 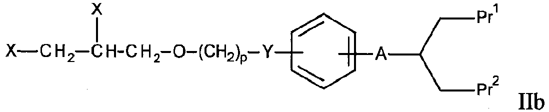 dans laquelle X, p et Y répondent aux définitions indiquées dans l'une quelconque des revendications précédentes, A représente un groupe CO, CHOH, CH.NH₂, CH.NHR, CH.NR₂, CH.ORCH.O.C(O)R, CH.NHC(O)R ou CH.N(C(O)R)₂, dans lequel chaque groupe R représente indépendamment un atome d'hydrogène ou un groupe alkyle, aryle ou alkylaryle, et soit chacun de Pr¹ et Pr² représente une molécule de protéine ou de peptide séparée, soit Pr¹ et Pr² représentent conjointement une protéine unique ou un peptide unique lié à deux points séparés.

11. Composé suivant la revendication 10, répondant à la formule générale : 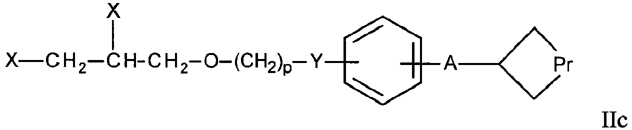 dans laquelle X, p, Y et A répondent aux définitions indiquées dans la revendication 10, et Pr représente une protéine unique ou un peptide unique lié à deux points séparés.

12. Composé suivant la revendication 11, dans lequel Pr représente une protéine unique ou un peptide unique lié à deux atomes de soufre dérivés d'une liaison disulfure dans ladite protéine ou ledit peptide, ou bien à deux résidus histidine présents dans un marqueur polyhistidine fixé à ladite protéine ou audit peptide.

13. Composé suivant la revendication 11 ou la revendication 12, dans lequel Pr représente un fragment Fab de IgG, le IFN-α, le IFN-β, ou un IFN consensus.

14. Procédé pour la préparation d'un composé suivant l'une quelconque des revendications 10 à 13, qui comprend la réaction d'un composé suivant l'une quelconque des revendications 1 à 9 avec une protéine ou un peptide ; et, facultativement, la réduction du groupe céto dans le conjugué résultant.

15. Composition pharmaceutique comprenant un composé suivant l'une quelconque des revendications 1 à 9, conjointement avec un support pharmaceutiquement acceptable, et contenant également facultativement un ingrédient actif supplémentaire.

16. Composé suivant l'une quelconque des revendications 1 à 9 ou composition suivant la revendication 15, pour une utilisation en thérapie.
